# EUROPEAN PATENT APPLICATION

(11) **EP 1 944 349 A1**
(43) Date of publication of application: **16.07.2008**
(21) Application number: 08250149.5
(22) Date of filing: 11.01.2008
(51) Int. Cl.: C09K 11/06, G02B 6/42

(54) **Organic electroluminescence and optical interconnect module**

(30) Priority: 11.01.2007 JP 2007003334
(71) Applicant: Fujikura, Ltd., Tokyo (JP); Shinshu University, Matsumoto-shi, Nagano 386-8567 (JP)
(72) Inventor: Fukuda, Takeshi, Sakura-shi, Chiba-ken (JP); Taniguchi, Yoshio c/o Shinsu University, Ueda-shi, Nagano-ken (JP)
(74) Representative: Walker, Edmund Mortimer

(57) **Abstract**

An organic electroluminescence which includes a first electrode layer, an organic layer having an emitting layer, and a second electrode layer, in which the emitting layer is made of at least one material selected from the group consisting of DPVBi, PBD, DSB, and BCzVB, and in which fluorescence lifetime of the emitting layer is equal to or less than 3.0 ns. A high-speed response organic electroluminescence, which is able to use as a light emitting element for optical communications, such as a optical interconnect module, can be provided.

## Description

### BACKGROUND OF THE INVENTION

### Field of the Invention

The present invention relates to an organic electroluminescence (hereafter referred to as "organic EL"). More specifically, the present invention relates to an organic electroluminescence of which a response speed is improved and which has an optical communication function; and to an optical interconnect module which uses the organic electroluminescence as a light emitting element.

### Description of Related Art

The basic structure of the organic EL is a structure which is deposited on a surface of a transparent glass substrate or of a transparent resin substrate in the following order: a first electrode layer (anode), an organic layer including emitting layer, and a second electrode layer (cathode).

The organic EL has advantages including high contrast ratio, wide field of viewing angle, and thickness. Therefore, the organic EL may be applied to fields, such as, the display field. However, in the general structure of the organic EL, the light emitting portion is formed on the drive transistor circuit. Therefore, the display using the organic EL may present a problem where the light emitted in the transistor part is absorbed or scattered and extraction efficiency to the outside turns worse. A structure called "top emission structure", which is constituted by depositing the cathode, organic layer, and anode on the glass substrate, is being examined to solve this problem.

The first electrode layer (anode) is made of a transparent conductive material represented by ITO (indium-tin oxide) or IZO (indium-zincoxide). The organic layer is comprised of multiple layers, such as the hole injection layer, the hole transport layer, the emitting layer, the electron transport layer, and the electron injection layer. The second electrode layer (cathode) is made of a metal such as Mg, Ag, Al, and Ca.

By now, research and development of the organic EL has been investigated for many luminous characteristics, such as, luminous efficiency, maximum luminance, and power consumption. Consequently, luminous characteristics have been improved drastically. For example, much research and development is performed on the phosphorescence materials of which the luminous efficiency is higher than that of the conventional fluorescence materials, cathode materials having a low work function, and the optimization of the carrier balance between the electron and the hole in the emitting layer. In addition, not only the conventional vacuum deposition method, but also de-vacuum processes performed by using a screen printing, gravure printing, and ink-jet printing, are being examined as manufacturing methods that are actually possible for lower production costs.

On the other hand, a light source for an optical interconnect module is expected to be a new application of the organic EL. The optical interconnect module has a structure in which a light emitting element and a light receiving element are provided on both ends of an optical fiber or a polymer optical waveguide. In the optical interconnect module, an electrical signal is converted into an optical signal by using the light emitting element and this light signal is sent to the light receiving element through the optical fiber or polymer optical waveguide. Finally, the optical signal is converted into an electrical signal with the light receiving element, thereby making communications.

Conventionally, as the optical interconnect module using the organic EL, for example, techniques as disclosed in Japanese Unexamined Patent Application Publication No. 2003-149541 and Japanese Unexamined Patent Application Publication No. 2003-14995 are proposed. By using these conventional techniques, the organic EL can be used as a light emitting element to transmit the light to the optical fiber or polymer optical waveguide. In addition, the organic EL can be formed directly on the substrate by using evaporation methods and the like, the substrate where the polymer optical waveguide, not having a high heat resistance, is formed. Therefore, the optical waveguide and organic EL can easily connected without precious optical axis adjustments and processing of the end surface of the optical waveguide.

Furthermore, it is possible that the optical waveguide and organic EL are formed integrally to accumulate monolithically. Therefore, the implementation process of the optical interconnect module can be largely shortened, and thus, being able to lower the production costs.

In addition, conventionally, as a method for improving a response speed of the organic EL, for example, techniques disclosed in Japanese Unexamined Patent Application Publication No. H5-29080, Japanese Unexamined Patent Application Publication No. 2003-243157, and Japanese Unexamined Patent Application Publication No. 2003-313553, are proposed. By using the method disclosed in Japanese Unexamined Patent Application Publication No. H5-29080, the response speed of the organic EL can be improved by lowering the capacitance of the organic EL. In Japanese Unexamined Patent Application Publication No. 2003-243157, the response speed of 100 MHz is realized by applying the voltage which is superposed the bias voltage and the pulse voltage. Furthermore, in Japanese Unexamined Patent Application Publication No. H5-29080, the response speed of the organic EL can be improved by providing a hole blocking layer next to the emitting layer.

However, in these conventional technologies, although the response speed of the organic EL can be improved to some extent, the response speed is still insufficient to apply to the practical optical interconnect module. The response speed of the organic EL for optical communications is required equal to or more than 100 MHz at the cutoff frequency and it is difficult to achieve the response speed of equal to or more than 100 MHz by the methods mentioned in these patent documents. Therefore, the organic EL having the response speed of equal to or more than 100 MHz is needed for optical communications.

The present invention was devised in view of the above circumstances, and has as a preferable object a feature of an organic EL which has a high response speed, and which is available to a light emitting element for optical communication devices, such as an optical interconnect module.

### BRIEF SUMMARY OF THE INVENTION

The first aspect of the present invention relates to an organic EL including a first electrode layer, an organic layer having an emitting layer, and a second electrode layer, in which the emitting layer is made of at least one material selected from the group consisting of 4,4-bis(2,2-ditolylvinyl)biphenyl (DPVBi),
2-(4-tert-butylphenyl)-5-(4-biphenylyl)-1,3,4-oxadiazole (PBD),
1,4-bis[2-[4-[*N*,*N*-di(*p*-tolyl)amino]phenyl]vinyl]benzene (DSB), and 4,4'-bis(9-ethyl-3-carbazovinylene)-1,1'-biphenyl (BCzVBi), and in which fluorescence lifetime of the emitting layer is equal to or less than 3.0 ns.

The second aspect of the present invention relates to an organic EL including a first electrode layer, an organic layer having an emitting layer, and a second electrode layer, in which the emitting layer is made of at least one material selected from the group consisting of 4,4-bis(2,2-ditolylvinyl)biphenyl (DPVBi), 2-(4-tert-butylphenyl)-5-(4-biphenylyl)-1,3,4-oxadiazole (PBD), and
1 ,4-bis[2-[ *4-[N* ,*N*-di(*p*-tolyl)amino]phenyl]vinyl]benzene (DSB), and in which fluorescence lifetime of the emitting layer is less than 0.6 ns.

The third aspect of the present invention relates to an organic EL including a first electrode layer, an organic layer having an emitting layer, and a second electrode layer, in which the emitting layer is made of materials where host materials and guest materials are mixed together, and in which the fluorescence lifetime of the emitting layer is less than 3.0 ns.

In the organic EL of the third aspect of the present invention, the host materials of the emitting layer may be 4,4'-bis(9-dicarbazolyl)-2,2'-biphenyl (CBP) or 2-(4-tert-butylphenyl)-5-(4-biphenylyl)-1,3,4-oxadiazole (PBD).

In the organic EL of the third aspect of the present invention, the guest materials of the emitting layer may be at least one material selected from the group consisting of 1,4-bis[2-[4-[*N*,*N*-di(*p*-tolyl)amino]phenyl]vinyl]benzene (DSB),
4,4-bis(2,2-ditolylvinyl)biphenyl (DPVBi),
4,4'-bis(9-ethyl-3-carbazovinylene)-1,1'-biphenyl (BCzVBi), and pelyrene.

In addition, the present invention relates to an optical interconnect module that uses the organic EL in the first, second and third aspects of the present invention as a light emitting element.

According to the present invention, the response speed of the organic EL can be improved by using the emitting layer materials having a short fluorescence lifetime.

In addition, since the intensity of the output light can be improved by using the emitting layer, in which more than 2 organic materials are mixed together, the organic EL having a high-output and high-speed response can be obtained.

Furthermore, since the optical interconnect module of the present invention uses the organic EL of the present invention having a high-speed response as a light emitting element, the organic EL can be formed directly on the substrate where a polymer optical waveguide, not having a high heat resistance, is formed. Therefore, the optical waveguide and organic EL can easily be connected without precious optical axis adjustments and processing of the end surface of the optical waveguide. Furthermore, it is possible that the optical waveguide and organic EL are formed integrally to accumulate monolithically. Therefore, the implementation process of the optical interconnect module can be largely shortened, thus being able to lower the production cost.

### BRIEF DESCRIPTION OF THE DRAWINGS

FIG. 1 is a block diagram of fluorescence lifetime used in Embodiment 1.
FIG. 2 is a graph showing the frequency dependence of the intensity of the fluorescence of the DSB thin film measured in Embodiment 1.
FIG. 3 is a graph showing the relationship between the cutoff frequency of four kinds of the thin film and fluorescence lifetime measured in Embodiment 1.

### DETAILED DESCRIPTION OF THE INVENTION

One embodiment of the organic EL of the present invention is characterized in that the emitting layer is made of at least one material selected from the group consisting of DPVBi, PBD, and DSB, and that fluorescence lifetime of the emitting layer is equal to or less than 3.0 ns.

Another embodiment of the organic EL of the present invention is characterized in that the emitting layer is made of materials where host materials and guest materials are mixed together, and that fluorescence lifetime of the emitting layer is equal to or less than 3.0 ns.

In this embodiment, the host materials of the emitting layer may be CBP or PBD. In addition, the guest materials of the emitting layer may be at least one material selected from the group consisting of DSB, BCzVBi, and pelyrene.

In the organic EL of the present invention, as long as the materials of the emitting layer have the above-mentioned characteristics, the other components are not limited and the organic EL may be configured equally with conventionally organic EL. For example, the organic EL of the present invention may have a basic structure which is deposited on a transparent substrate, such as a glass substrate or a transparent resin substrate in the following order: a first electrode layer (cathode) made of a transparent conductive material such as ITO and IZO, an organic layer including an emitting layer, and a second electrode layer (anode) consisting of a metal thin film. The organic EL may have a "top emission structure" laminated in order of a cathode, an organic layer, and an anode on a glass substrate.

The response speed of the organic EL is affected not only by fluorescence lifetime but also by other parameters which are the carrier movement time from the electrode to the emitting layer, the carrier injection time at organic and metal interface. As explained in detail later, the relationship between fluorescence lifetime and the response speed of the organic EL is clarified for the first time by the present invention. In the Embodiment 1, the value of fluorescence lifetime necessary to realize a cutoff frequency of equal to or more than 100 MHz is specifically estimated by directly evaluating only the effect of fluorescence lifetime on the response speed.

### [Embodiment 1]

In order to directly examine the relationship between fluorescence lifetime of the organic emitting layer and the response speed of the organic EL, the value of fluorescence lifetime necessary to realize the cutoff frequency of 50 MHz and 100 MHz was evaluated by comparing measurement results of fluorescence lifetime and measurement results of the frequency dependence of the intensity of the fluorescence when an organic emitting material was excited by a modulated violet laser diode. Fluorescence lifetime was measured by using a streak camera.

The measurement results of fluorescence lifetime of the organic thin film using the present invention method are shown. Four kinds of the organic thin films, (1) DSB, (2) tris(8-hydroxyquinoline) aluminum (Alq₃), (3) Alq₃ doped with 4-(dicyanomethylene) 2-methyl-6-(julolidine-4-yl-vinyl)-4H-pyran (DCM2), and (4) Alq₃ doped with 5,6,11,12-tetra phenyl-tetracene at 0.5 percent by mass (rubrene), were deposited onto the glass substrate at a film thickness of 100 nm.

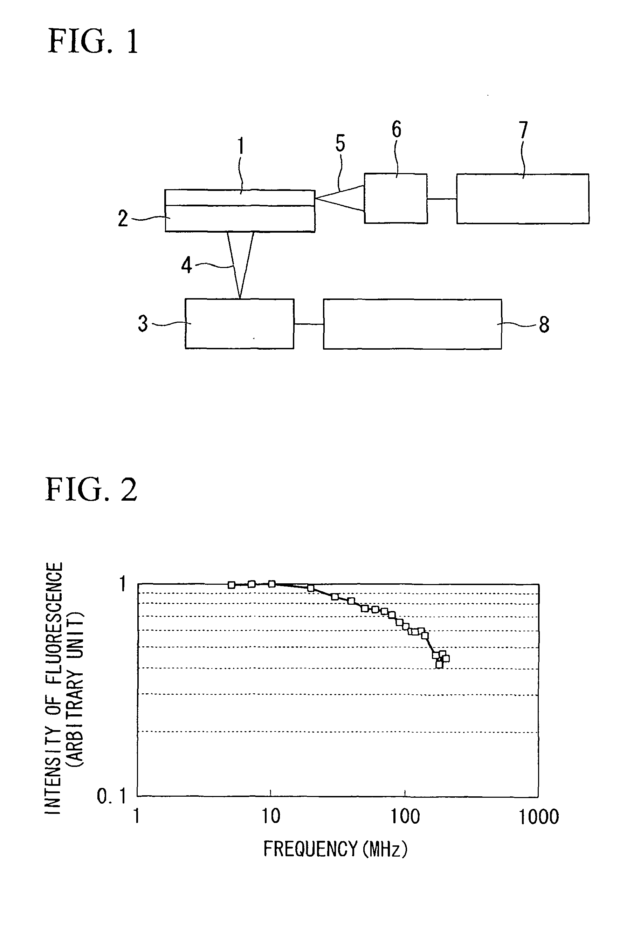

The block diagram of the measurement device of fluorescence lifetime is shown in FIG. 1. In FIG. 1, symbol 1 is an organic thin film, 2 is a glass substrate, 3 is a violet laser diode, 4 is a laser beam, 5 is a fluorescence, 6 is a photodiode (photo detector), 7 is an oscilloscope, and 8 is a signal generator. The sine wave voltage was impressed to the violet laser diode 3 having a center wavelength of 405 nm by using the signal generator 8 (Kenwood SG-7200^{™}, made by the Kenwood Corporation), and a light of which the intensity is modulated was generated. The fluorescence 5 was generated by radiating the intensely-modulated light on the organic thin film 1. The generated fluorescence 5 was converted into an electrical signal by the photo detector 6 ( S-5343™ made by the Hamamatsu Photonics Company), and the intensity of the electrical signal was measured with the oscilloscope 7 (DL1740™ made by the Yokokawa Electric Company). Here, the frequency of the sine wave voltage generated by the signal generator 8 was changed and the intensity of the fluorescence 5 was measured at each frequency.

The frequency dependence of the intensity of the fluorescence 5 of the DSB thin film is shown in FIG. 2. Here, the modulation frequency was changed from a range of 1 MHz to 200 MHz. In FIG. 2, the axis of ordinate and axis of abscissa are each displayed with a logarithm. In addition, the frequency at which the intensity of the fluorescence was reduced to 50% was determined as a cutoff frequency. In the case of DSB thin film, the cutoff frequency became 160 MHz. Fluorescence lifetime was measured with the streak camera (FESCA-200™ made by the Hamamatsu Photonics Company) in the same measurement object, and it became 0.2 ns. In other words, it is obvious that the cutoff frequency of 160 MHz correspond to fluorescence lifetime of 0.2 ns.

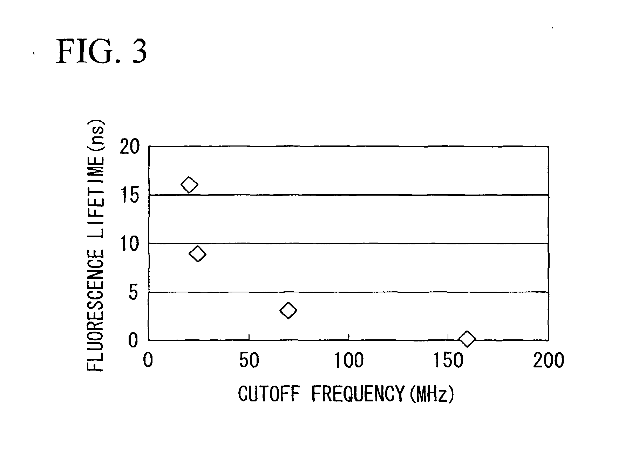

The relationship between the cutoff frequency of above-mentioned four kinds of the thin film and fluorescence lifetime is shown in FIG. 3. Here, the value of fluorescence lifetime was measured by using the streak camera (FESCA-200™ made by the Hamamatsu Photonics Company). There is a clear relationship between fluorescence lifetime and the cutoff frequency. Therefore, it is possible to estimate fluorescence lifetime from the cutoff frequency by using the result of FIG. 3. From this result, it is understood that fluorescence lifetime of equal to or less than 3.0 ns is necessary to realize the response speed of equal to or more than 50 MHz, and that fluorescence lifetime of equal to or less than 0.6 ns is necessary to realize the response speed of equal to or more than 100 MHz.

### [Embodiment 2]

(1) Alq₃, (2) DSB, (3) BCzVBi, (4) DPVBi, (5) Perylene, (6) CBP, and (7) PBD were each evaporated at a film thickness of 100nm by using a disposition device of resistance wire heating type.

Fluorescence lifetime of the manufactured thin film was measured with the streak camera and the results are shown in table 1. In the measurement of fluorescence lifetime, the second harmonics ofTi: sapphire laser was used as an excitation light and the wavelength of the excitation light was set to 380 nm. The excitation light was radiated on the organic thin film, and the generated fluorescence was received by the streak camera (FESCA-200™ made by the Hamamatsu Photonics Company). Fluorescence lifetime was estimated from a change in time of the intensity of the fluorescence.

**[Table 1]**

| Material | Alq₃ | CBP | DSB | DPVBi | PBD | BCzVBi | Perylene |
|---|---|---|---|---|---|---|---|
| Fluorescence Lifetime (ns) | 16.0 | 3.9 | 0.2 | 0.5 | 1.7 | 0.6 | 9.3 |

As the results shown in Table 1, fluorescence lifetime became equal to or less than 3.0 ns when the four kinds of materials of DSB, BCzVBi, PBD, and DPVBi were used. Furthermore, fluorescence lifetime became equal to or less than 0.6 ns when the three kinds of materials of DSB, BCzVBi, and DPVBi were used.

### [Embodiment 3]

In Table 2, the measurement results of fluorescence lifetime in six kinds of the organic thin films, Alq₃ doped with rubrene, CBP doped with DSB, CBP doped with BCzVBi, CBP doped with DPVBi, PBD doped with BCzVBi, and PBD doped with Perylene, are shown. Here, the density of the dopants (rubrene, DSB, BCzVBi, DPVBi, and Perylene) was set to 0.5 percent by mass in all samples.

**[Table 2]**

| | | | |
|---|---|---|---|
| Material | Alq₃ doped with rubrene | CBP doped with DSB | CBP doped with DPVBi |
| Fluorescence Lifetime (ns) | 9.2 | 1.4 | 1.6 |
| Material | CBP doped with BCzVBi | PBD doped with BCzVBi | PBD doped with Perylene |
| Fluorescence Lifetime (ns) | 1.5 | 0.8 | 1.6 |

As the result shown in Table 2, in the case when two materials are mixed (doped), in the five kinds of combinations of materials, that is, CBP doped with DSB, CBP doped with BCzVBi, CBP doped with DPVBi, PBD doped with BCzVBi, and PBD doped with perylene, fluorescence lifetime became equal to or less than 3.0 ns. When two materials were mixed (doped), it is possible to prevent prolongation of fluorescence lifetime due to concentration quenching. Therefore, fluorescence lifetime may be shortened as a result. Furthermore, by holding down this concentration quenching, it is known that other characteristics such as luminous efficiency are also improved. Consequently, the organic EL of the present invention has the structure suitable for communications.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims as interpreted by the description and drawings.

## Claims

1. An organic electroluminescence comprising:
a first electrode layer;
an organic layer (1) having an emitting layer; and
a second electrode layer, **characterized in that**:
the emitting layer is made of at least one material selected from the group consisting of 4,4-bis(2,2-ditolylvinyl)biphenyl,
2-(4-tert-butylphenyl)-5-(4-biphenylyl)-1,3,4-oxadiazole,
1,4-bis[2-[4-[*N*,*N*-di(*p*-tolyl)amino]phenyl]vinyl]benzene, and
4,4'-bis(9-ethyl-3-carbazovinylene)-1,1'-biphenyl, and
the emitting layer has a fluorescence lifetime equal to or less than 3.0 ns.

2. An organic electroluminescence comprising:
a first electrode layer;
an organic layer (1) having an emitting layer; and
a second electrode layer, **characterized in that**:
the emitting layer is made of at least one material selected from the group consisting of 4,4-bis(2,2-ditolylvinyl)biphenyl, 2-(4-tert-butylphenyl)-5-(4-biphenylyl)-1,3,4-oxadiazole, and 1,4-bis[2-[4-[*N*,*N*-di(*p*-tolyl)amino]phenyl]vinyl]benzene, and
the emitting layer has a fluorescence lifetime equal to or less than 0.6 ns.

3. An organic electroluminescence comprising:
a first electrode layer;
an organic layer having an emitting layer; and
a second electrode layer, **characterized in that**:
the emitting layer is made of materials where host materials and guest materials are mixed together, and
the emitting layer has a fluorescence lifetime equal to or less than 3.0 ns.

4. The organic electroluminescence according to claim 3, wherein the host material of the emitting layer is 4,4'-bis(9-dicarbazolyl)-2,2'-biphenyl or 2-(4-tert-butylphenyl)-5-(4-biphenylyl)-1,3,4-oxadiazole.

5. The organic electroluminescence according to claim 3, wherein the guest materials of the emitting layer is at least one material selected from the group consisting of 1,4-bis[2-[4-[*N*,*N*-di(*p*-tolyl)amino]phenyl]vinyl]benzene, 4,4-bis(2,2-ditolylvinyl)biphenyl, 4,4'-bis(9-ethyl-3-carbazovinylene)-1,1'-biphenyl, and pelyrene.

6. The organic electroluminescence according to claim 4, wherein the guest materials of the emitting layer is at least one material selected from the group consisting of 1,4-bis[2-[4-[*N,N*-di(*p*-tolyl)amino]phenyl]vinyl]benzene, 4,4-bis(2,2-ditolylvinyl)biphenyl, 4,4'-bis(9-ethyl-3-carbazovinylene)-1,1'-biphenyl, and pelyrene.

7. An optical interconnect module having an organic electroluminescence according to claim 1 as a light emitting element.

8. An optical interconnect module having an organic electroluminescence according to claim 2 as a light emitting element.

9. An optical interconnect module having an organic electroluminescence according to claim 3 as a light emitting element.
